# EUROPÄISCHE PATENTSCHRIFT

(11) **EP 1 339 658 B1**
(45) Veröffentlichungstag und Bekanntmachung des Hinweises auf die Patenterteilung: **24.10.2007**
(21) Anmeldenummer: 01996522.7
(22) Anmeldetag: 15.11.2001
(51) Int. Cl.: C07C 11/167, C07C 7/04

(54) **VERFAHREN UND VORRICHTUNG ZUR DESTILLATIVEN GEWINNUNG VON 1,3-REINBUTADIEN AUS 1,3-ROHBUTADIEN**
METHOD AND DEVICE FOR OBTAINING 1,3 PURE BUTADIENE FROM 1,3 RAW BUTADIENE BY DISTILLATION
PROCEDE ET DISPOSITIF POUR OBTENIR DU 1,3-BUTADIENE PUR A PARTIR DE 1,3-BUTADIENE BRUT PAR DISTILLATION

(30) Priorität: 16.11.2000 DE 10056841
(43) Veröffentlichungstag der Anmeldung: 03.09.2003
(73) Patentinhaber: BASF Aktiengesellschaft, 67056 Ludwigshafen (DE)
(72) Erfinder: BOHNER, Gerd, 69254 Malsch (DE); KINDLER, Klaus, 67376 Harthausen (DE); PAHL, Melanie, 68167 Mannheim (DE); KAIBEL, Gerd, 68623 Lampertheim (DE)
(74) Vertreter: Isenbruck, Günter
(86) Internationale Anmeldenummer: PCT/EP2001/013235
(87) Internationale Veröffentlichungsnummer: WO 2002/040434

(56) Entgegenhaltungen:
- EP-A- 0 141 356
- DE-A- 1 926 085
- DE-A- 3 302 525

## Beschreibung

Die Erfindung betrifft ein Verfahren zur destillativen Gewinnung von 1,3-Reinbutadien aus 1,3-Rohbutadien.

1,3-Butadien wird großtechnisch in der Regel aus dem sogenannten C₄-Schnitt gewonnen, d. h. aus einem Gemisch von Kohlenwasserstoffen, worin die C₄-Kohlenwasserstoffe, insbesondere 1-Buten, i-Buten sowie 1,3-Butadien überwiegen. Neben geringen Mengen an C₃- und C₅-Kohlenwasserstoffen enthält der C₄-Schnitt in der Regel Butine, insbesondere 1-Butin (Ethylacetylen) und Butenin (Vinylacetylen). Hierbei fällt zunächst ein sogenanntes 1,3-Rohbutadien an, d. h. ein Gemisch, das etwa 89 bis 99,5 Gew.-% 1,3-Butadien, Rest Verunreinigungen, enthält. Dieses muss anschließend, um den Spezifikationsanforderungen zu entsprechen, zu sogenanntem 1,3-Reinbutadien weiter destillativ gereinigt werden. Die Spezifikationsanforderungen für 1,3-Reinbutadien sehen insbesondere einen Mindestgehalt an 1,3-Butadien von 99,6 Gew.-% und einen maximal zulässigen Gehalt an Propin von 10 ppm und an 1,2-Butadien von 20 ppm vor.

Die Gewinnung von 1,3-Rohbutadien aus dem C₄-Schnitt ist wegen der geringen Unterschiede in den relativen Flüchtigkeiten der Komponenten ein kompliziertes destillationstechnisches Problem und wird daher in der Regel durch sogenannte Extraktivdestillation durchgeführt.

In besonders vorteilhafter Weise können auch die acetylenischen C₄-Verunreinigungen, insbesondere Ethylacetylen und Vinylacetylen in das Wertprodukt 1,3-Butadien übergeführt werden, wenn der Extraktivdestillation eine Selektivhydrierung vorgeschaltet wird, wie beispielsweise in US 4,277,313 beschrieben oder besonders vorteilhaft indem Extraktivdestillation und Selektivhydrierung in heterogener Katalyse in einer einzigen Kolonne, vorzugsweise einer Trennwandkolonne oder in thermisch gekoppelten Kolonnen durchgeführt werden. Ein derartiges Verfahren ist in der deutschen Patentanmeldung 10022465.2 beschrieben. In den bekannten Verfahren zur Extraktivdestillation bzw. zur Extraktivdestillation und Selektivhydrierung, beispielsweise gemäß DE 10022465.2, fällt jedoch zunächst ein 1,3-Butadien an, das den Spezifikationsanforderungen noch nicht genügt, sogenanntes 1,3-Roh-Butadien.

Aus EP-A 0 141 356 ist ein Verfahren zur Gewinnung von beispielsweise 1,3-Butadien aus einem C₄-Kohlenwasserstoffgemisch mittels Extraktivdestillation bekannt, wonach man das C₄-Kohlenwasserstoffgemisch in eine die schwerer löslichen Kohlenwasserstoff enthaltendes Destillat, einen 1,3-Butadien enthaltenden Produktstrom und ein die leichter löslichen Kohlenwasserstoffe enthaltenden Strom auftrennt und bei dem man die Extraktivdestillation unter Verwendung einer Destillatabzugszone (= Trennzone C), eine Zulaufzone für das Ausgangs-C₄-Kohlenwasserstoffgemisch (= Trennzone A), eine Produktabzugszone für den Produktstrom (= Trennzone B) und eine Ausstreifzone, in der das selektive Lösungsmittel zurückgewonnen und aus der die leichter löslichen Kohlenwasserstoffe enthaltende Strom abgezogen wird (= Trennzone D) und wobei man
a) das Ausgangs-C₄-Kohlenwasserstoffgemisch der Trennzone A zuführt,
b) das der Extraktivdestillation zuzuführende selektive Lösungsmittel im oberen Teil der Trennzone C zugibt,
c) das im unteren Teil der Trennzone C erhaltene geladene selektive Lösungsmittel zu einem Teil im oberen Teil der Trennzone A und zum anderen Teil im oberen Teil der Trennzone B zuführt,
d) das im unteren Teil der Trennzone A erhaltene beladene selektive Lösungsmittel und das im unteren Teil der Trennzone D erhaltene beladene selektive Lösungsmittel der Trennzone D zuführt, aus der der die leichter löslichen Kohlenwasserstoffe enthaltende Strom und ein Strom des von den Kohlenwasserstoffen ganz oder teilweise befreiten selektiven Lösungsmittels abgezogen werden,
e) aus der Trennzone D einen 1,3-Butadien enthaltenden Produktstrom und
f) aus der Trennzone C das Destillat abzieht.

Die parallelen Trennzonen A und B können apparativ in und demselben Kolonnenmantel untergebracht werden, wobei zur Trennung der beiden Zonen A und D voneinander ein senkrechtes Trennblech eingebaut ist.

Die destillative Gewinnung von 1,3-Reinbutadien aus 1,3-Rohbutadien erfolgt im Stand der Technik zweistufig: In einer ersten Stufe wird bei einem Kolonnendruck von etwa 7 bar ein Gemisch von überwiegend Propin und Propadien als Kopfprodukt abgezogen, in einer zweiten nachgeschalteten Destillationskolonne werden bei einem Druck von etwa 4,5 bar als Sumpfprodukt 1,2-Butadien und C₅-Kohlenwasserstoffe abgetrennt. Das im 1,3-Rohbutadien vorhandene cis-2-Butin erscheint etwa hälftig am Kopf und am Sumpf der zweiten Destillationskolonne. Das Wertprodukt 1,3-Reinbutadien wird als Kopfprodukt der zweiten Destillationskolonne entnommen.

Aus EP-B 284 971 ist es auch bekannt, die beiden Destillationskolonnen thermisch gekoppelt auszugestalten. Auch nach dem Verfahren der EP-B 284 971 werden die beiden Destillationskolonnen bei unterschiedlichen Drücken betrieben, müssen somit mit jeweils eigenem Verdampfer und Kondensator ausgerüstet werden, mit der Folge einer lediglich geringfügigen Verringerung des Energiebedarfs gegenüber der Variante mit zwei nicht thermisch gekoppelten Destillationskolonnen.

Alle bekannten Verfahrensvarianten zur destillativen Gewinnung von 1,3-Reinbutadien aus 1,3-Rohbutadien waren von der Annahme ausgegangen, dass eine Betriebsweise bei zwei unterschiedlichen Drücken, mit niedrigerem Druck in der zweiten Destillationskolonne gegenüber der ersten Destillationskolonne im Hinblick auf die thermischen Empfindlichkeit der zur Polymerisation neigenden Diene sowie die bessere Kondensierbarkeit des Propin/Propadien-Gemisches am Kopf der ersten Destillationskolonne zwingend erforderlich sei.

Demgegenüber war es Aufgabe der Erfindung, ein verbessertes Verfahren und eine Vorrichtung zur destillativen Gewinnung von 1,3-Reinbutadien aus 1,3-Rohbutadien zur Verfügung zu stellen, das, bei Einhaltung der geforderten Spezifikationen, wirtschaftlicher ist, insbesondere bezüglich der Investitions- und Energiekosten.

Die Lösung geht aus von einem Verfahren der destillativen Gewinnung 1,3-Reinbutadien aus 1,3-Rohbutadien.

Die Erfindung ist dadurch gekennzeichnet, dass das Verfahren in einer Trennwandkolonne durchgeführt wird, in der eine Trennwand in Kolonnenlängsrichtung unter Ausbildung eines oberen gemeinsamen Kolonnenbereichs, eines unteren gemeinsamen Kolonnenbereichs, eines Zulaufteils und eines Entnahmeteils angeordnet und dass die Verweilzeit im Sumpfverdampfer und dem dazu gehörigen Rohrleitungssystem auf 1 bis 15 Minuten begrenzt ist.

Es wurde überraschend gefunden, dass die destillative Gewinnung von 1,3-Reinbutadien aus 1,3-Rohbutadien entgegen der Annahme, dass eine zweistufige Betriebsweise mit unterschiedlichen Drücken erforderlich sei, in einer einzigen Kolonne, und zwar einer Trennwandkolonne und somit bei einheitlichem Druck durchgeführt werden kann.

Das Verfahren wird erfindungsgemäß in einer Trennwandkolonne durchgeführt. Trennwandkolonnen sind Destillationskolonnen mit senkrechten Trennwänden, die in Teilbereichen eine Quervermischung von Flüssigkeits- und Brüdenströmen verhindern. Die Trennwand, die aus einem ebenen Blech besteht, unterteilt die Kolonne in Längsrichtung in deren mittlerem Bereich in einen Zulaufteil und einen Entnahmeteil. Das aufzutrennende Gemisch, das 1,3-Rohbutadien, wird dem Zulaufteil zugeführt und das Produkt, das 1,3-Reinbutadien wird aus dem Entnahmeteil abgezogen.

Das Verfahren wird in der Regel kontinuierlich durchgeführt.

Die Trennwandkolonne ist, wie in der Regel jede Destillationskolonne, mit einem Sumpfverdampfer und einem Kondensator am Kolonnenkopf ausgestattet.

Im Verfahren der vorliegenden Erfindung wird in vorteilhafter Weise die Verweilzeit im Sumpfverdampfer und dem zugehörigen Rohrleitungssystem auf 3 bis 6 Minuten, begrenzt. Dadurch wird trotz der Polymerisationsanfälligkeit des Gemisches mit zahlreichen ungesättigten Komponenten ein störungsfreier Betrieb der Anlage, insbesondere nur geringfügige oder keine Verschmutzungen gewährleistet.

In einer bevorzugten Verfahrensvariante wird das Flüssigkeitsrücklaufverhältnis am oberen Ende der Trennwand auf den Zulauf- bzw. Entnahmeteil der Kolonne im Verhältnis von 1:1,3 bis 2,2, bevorzugt im Verhältnis von 1:1,6 bis 1,9 geregelt. Dies erfolgt bevorzugt in der Weise, dass die Flüssigkeit am oberen Ende der Trennwand gesammelt und über eine Regelungs- oder Stellvorrichtung im genannten Verhältnis auf den Zulauf- bzw.

Entnahmeteil der Kolonne aufgegeben wird. Dadurch wird ein niedrigerer Energieverbrauch gewährleistet.

In einer weiteren bevorzugten Verfahrensvariante zusätzlich oder alternativ zur Regelung des Flüssigkeitsrücklaufverhältnisses am oberen Ende der Trennwand das Mengenverhältnis der Brüdenströme am unteren Ende der Trennwand auf den Zulaufteil beziehungsweise auf den Entnahmeteil der Kolonne im Verhältnis von 1:0,7 bis 1,3, bevorzugt im Verhältnis von 1:0,95 bis 1,1, eingestellt. Dies erfolgt bevorzugt durch Wahl der Trenneinbauten und/oder durch den zusätzlichen Einbau druckverlusterzeugender Einbauten, beispielsweise von Blenden oder durch eine Mengenregelung der Brüdenströme.

Das erfindungsgemäße Verfahren wird bevorzugt bei einem Druck im Kolonnenkopf von 2 bis 10 bar, bevorzugt von 4 bis 7 bar, durchgeführt.

Bevorzugt ist im oberen gemeinsamen Kolonnenbereich eine Temperaturregelung vorgesehen, mit einer Messstelle unterhalb des obersten theoretischen Bodens, bevorzugt auf dem dritten theoretischen Boden von oben gezählt, die als Stellgröße den Destillatstrom, das Rücklaufverhältnis oder bevorzugt die Rücklaufmenge, nutzt. Dadurch wird ein stabiler Betrieb der Kolonne gewährleistet mit der Folge einer weiteren Verbesserung der erzielbaren Produktreinheit.

In einer weiteren Verfahrensvariante ist zusätzlich oder alternativ eine Temperaturregelung im unteren Kolonnenbereich vorgesehen mit einer Messstelle oberhalb des untersten theoretischen Bodens, bevorzugt auf dem zweiten theoretischen Boden von unten gezählt, die als Stellgröße die Sumpfentnahmemenge nutzt. Durch diese zusätzliche Maßnahme wird eine weitere Verbesserung des stabilen Kolonnenbetriebs erreicht.

Darüber hinaus ist es zusätzlich oder alternativ möglich, eine Standregelung am Kolonnensumpf vorzusehen, die als Stellgröße die Seitenentnahmemenge nutzt.

Die im erfindungsgemäßen Verfahren eingesetzte Trennwandkolonne weist eine Anzahl von theoretischen Böden von etwa 40 bis 70, bevorzugt von 50 bis 60 auf.

Bevorzugt ist die Zulaufstelle für das 1,3-Rohbutadien auf einem theoretischen Boden zwischen dem 20. bis 40., bevorzugt zwischen dem 25. bis 35. theoretischen Boden angeordnet.

Die Seitenentnahmestelle für das 1,3-Reinbutadien erfolgt bevorzugt von einem theoretischen Boden zwischen dem 25. bis 50., bevorzugt zwischen dem 33. bis 40. theoretischen Boden.

Die Trennwand ist in der Kolonne zwischen dem 10. und 60., bevorzugt zwischen dem 15. und 53. theoretischen Boden, bevorzugt mittig, angeordnet.

Bezüglich der trennwirksamen Einbauten gibt es grundsätzlich keine Einschränkungen; bevorzugt sind geordnete Packungen oder Böden vorgesehen.

In einer bevorzugten Ausgestaltung sind die Böden so ausgelegt, insbesondere bezüglich der Wehrhöhen, dass die Verweilzeit in der Kolonne 15 Minuten, bevorzugt 10 Minuten, nicht überschreitet.

Die Erfindung wird im Folgenden anhand einer Zeichnung und eines Ausführungsbeispiels näher erläutert.

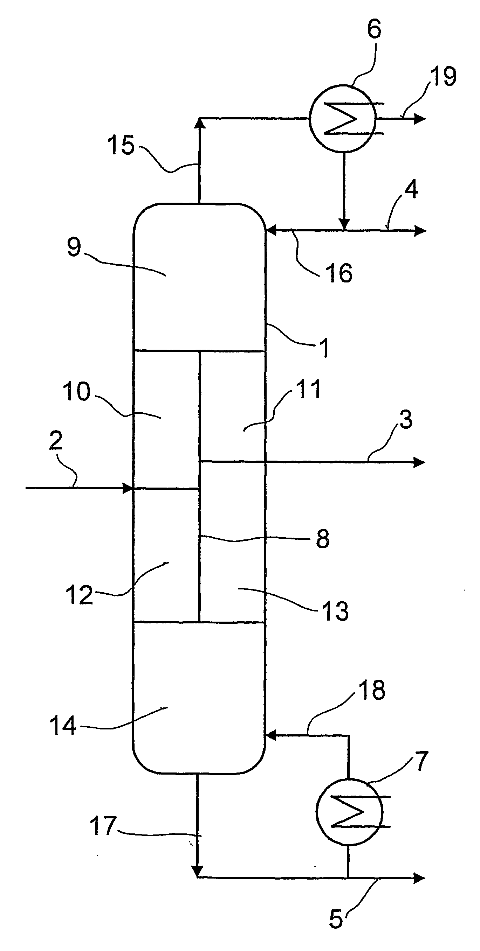

Die Zeichnung zeigt in der einzigen Figur eine Trennwandkolonne 1 mit Trennwand 8, die die Trennwandkolonne 1 in einen gemeinsamen oberen Kolonnenbereich 9, einen Zulaufteil 10, 12, mit Verstärkungsteil 10 und Abtriebsteil 12 einen Entnahmeteil 11,13 mit einem Abtriebsteil 11 und einem Verstärkungsteil 13 sowie einen gemeinsamen unteren Kolonnenbereich 14 unterteilt. Das 1,3-Rohbutadien 2 tritt zwischen den Kolonnenteilen 10 und 12 in die Trennwandkolonne 1 ein. Das 1,3-Reinbutadien 3 wird zwischen den Kolonnenteilen 11 und 13, bevorzugt in flüssiger Form, entnommen. Der am Kolonnenkopf anfallende Brüdenstrom 15 wird im Kondensator 6, der gegebenenfalls durch einen Nachkühler ergänzt wird, teilweise kondensiert und in den Rücklaufstrom 16 sowie den Destillatstrom 4 aufgeteilt. Der nicht kondensierte Anteil aus dem Kondensator 6 enthält die leichtsiedenden Verunreinigungen und wird dampfförmig als Strom 19 abgezogen. Am unteren Kolonnenende wird die Flüssigkeit 17 in einem Verdampfer 7 teilweise verdampft und über die Rohrleitung 18 in die Kolonne zurückgeführt. Ein Teilstrom 5, der die schwersiedenden Verunreinigungen enthält, wird abgezogen. Der Verdampfer 7 kann als Naturumlaufverdampfer oder als Zwangsumlaufverdampfer ausgebildet sein, im letzteren Fall ist zusätzlich eine Umlaufpumpe für den Flüssigkeitsstrom 17 erforderlich. Besonders vorteilhaft hinsichtlich der Vermeidung unerwünschter Polymerisationsreaktionen ist es anstelle des Zwangsumlaufverdampfers einen Fallfilmverdampfer einzusetzen, da mit dieser Bauart die kürzesten Verweilzeiten möglich sind. Zur Verringerung der Verweilzeit der Flüssigkeit im Verdampfersystem ist es günstig, die Standhaltung nicht in der unteren Kolonnenhaube, sondern in der Zulaufleitung der Flüssigkeit 17 anzuordnen.

### Beispiel:

Ein 1,3-Rohbutadienstrom von 11.027 kg/h mit einer Temperatur von 43,8°C wurde flüssig auf dem 30. theoretischen Boden einer Trennwandkolonne 1 mit insgesamt 55 theoretischen Böden eingespeist. Das 1,3-Rohbutadien wies die folgende Zusammensetzung auf:

| | |
|---|---|
| Propin | 800 ppm |
| n-Butan | 9 ppm |
| i-Butan | 17 ppm |
| n-Buten | 28 ppm |
| i-Buten | 49 ppm |
| trans-2-Buten | 13 ppm |
| cis-2-Buten | 0,27 Gew.-% |
| 1,3 Butadien | 99,44 Gew.-% |
| 1,2 Butadien | 0,14 Gew.-% |
| 1-Butin | 49 ppm |
| C4-Acetylene | 82 ppm |
| C5-Komponenten | 48 ppm |
| Wasser | 405 ppm. |

Die Trennwand 8 erstreckte sich vom 20. bis zum 51. theoretischen Boden. Die Seitenentnahme 3 erfolgte auf dem 37. theoretischen Boden. Der Betrieb der Kolonne erfolgte bei einem Kopfdruck von 5,5 bar und einem Sumpfdruck von 5,75 bar.

Am Kopf der Kolonne wurde bei einer Temperatur von 40°C kondensiert. Aus dem Kondensator 6 wurde ein dampfförmiger leichtsiederhaltiger Strom 19 von 26,4 kg/h abgezogen. Aus dem kondensierten Strom wurde ein Teilstrom 4 von 4,4 kg/h abgezogen. Die hochsiedenden Verunreinigungen 5 wurden am Kolonnensumpf in einem Mengenstrom von 28 kg/h bei einer Temperatur von 62°C entnommen. Am Seitenabzug wurde das Wertprodukt 1,3-Reinbutadien bei einer Temperatur von 49,7°C flüssig in einer Menge von 10.968,5 kg/h mit einem Gehalt an 1,3-Butadien von 99,76 Gew.% erhalten. Die handelsüblichen Spezifikationen für Propin wurden mit 10 ppm und für 1,2-Butadien mit 20 ppm eingehalten. Die Destillationsausbeute für 1,3-Butadien lag bei über 99,8 %.

Das Aufteilungsverhältnis für die Flüssigkeit am oberen Ende der Trennwand 8 betrug 1:1,8 zwischen Zulaufteil und Entnahmeteil. Am unteren Ende der Trennwand erfolgte die Aufteilung des Brüdenstroms im Verhältnis 1:1 auf den Zulauf- bzw. den Entnahmeteil. Die Heizleistung betrug 4.778 kW.

Durch das erfindungsgemäße Verfahren konnte die Destillation von 1,3-Rohbutadien zu 1,3-Reinbutadien bei einer Jahreskapazität von 90.000 t unter Einhaltung der geforderten Spezifikationen mit einer Investitionskosteneinsparung von 20% und einer Energiekosteneinsparung von 16% gegenüber dem herkömmlichen zweistufigen Destillationsverfahren durchgeführt werden.

## Patentansprüche

1. Verfahren zur destillativen Gewinnung von 1,3-Reinbutadien, enthaltend mindestens 99,6 Gew.-% 1,3-Butadien, maximal 10 ppm Propin und maximal 20 ppm 1,2-Butadien, aus 1,3-Rohbutadien, enthaltend etwa 89 bis 99,5 Gew.-% 1,3-Butadien, Rest Verunreinigungen, **dadurch gekennzeichnet, dass** das Verfahren in einer Trennwandkolonne (1) durchgeführt wird, in der eine Trennwand (8) in Kolonnenlängsrichtung unter Ausbildung eines oberen gemeinsamen Kolonnenbereichs (9), eines unteren gemeinsamen Kolonnenbereichs (14), eines Zulaufteils (10,12) und eines Entnahmeteils (11,13) angeordnet ist, und dass man die Verweilzeit im Sumpfverdampfer (7) und dem zugehörigen Rohrleitungssystem auf 1 bis 15 Minuten begrenzt.

2. Verfahren gemäß Anspruch 1, **dadurch gekennzeichnet, dass** man die Verweilzeit im Sumpfverdampfer (7) und dem dazugehörigen Rohrleitungssystem auf 3 bis 6 Minuten begrenzt.

3. Verfahren nach Anspruch 1 oder 2, **dadurch gekennzeichnet, dass** man das Flüssigkeitsrücklaufverhältnis am oberen Ende der Trennwand (8) auf den Zulauf-(10) bzw. den Entnahmeteil (11) der Kolonne im Verhältnis von 1 zu 1,3 bis 2,2 bevorzugt.

4. Verfahren nach einem der Ansprüche 1 bis 3, **dadurch gekennzeichnet, dass** man das Mengenverhältnis der Brüdenströme am unteren Ende der Trennwand (8) auf den Zulaufteil (12) bzw. den Entnahmeteil (13) der Kolonne im Verhältnis von 1 zu 0,7 bis 1,3, bevorzugt im Verhältnis von 1 zu 0,95 bis 1,1, einstellt.

5. Verfahren nach einem der Ansprüche 1 bis 4, **gekennzeichnet durch** einen Druck am Kolonnenkopf von 2 bis 10 bar, bevorzugt von 4 bis 7 bar.

6. Verfahren nach einem der Ansprüche 1 bis 5, **gekennzeichnet durch** eine Temperaturregelung im oberen gemeinsamen Kolonnenbereich, mit einer Messstelle unterhalb des obersten theoretischen Bodens, bevorzugt auf dem 3. theoretischen Boden von oben gezählt, die als Stellgröße den Destillatstrom, das Rücklaufverhältnis oder bevorzugt die Rücklaufmenge nutzt.

7. Verfahren nach einem der Ansprüche 1 bis 6, **gekennzeichnet durch** eine Temperaturregelung im unteren gemeinsamen Kolonnenbereich mit einer Messstelle oberhalb des untersten theoretischen Bodens, bevorzugt auf dem 2. theoretischen Boden von unten gezählt, die als Stellgröße die Sumpfentnahmemenge nutzt.

8. Verfahren nach einem der Ansprüche 1 bis 7, **gekennzeichnet durch** eine Standregelung am Kolonnensumpf, die als Stellgröße die Seitenentnahmemenge nutzt.

9. Verfahren nach einem der Ansprüche 1 bis 8, **gekennzeichnet durch** eine Anzahl von theoretischen Böden von etwa 40 bis 70, bevorzugt von 50 bis 60 in der Trennwandkolonne (1).

10. Verfahren nach Anspruch 9, **gekennzeichnet durch** die Anordnung der Zulaufstelle (2) für das 1,3-Rohbutadien auf einem theoretischen Boden zwischen dem 20. bis 40., bevorzugt zwischen dem 25. bis 35. theoretischen Boden.

11. Verfahren nach einem der Ansprüche 9 oder 10, **gekennzeichnet durch** eine Seitenentnahmestelle (3) für das 1,3-Reinbutadien von einem theoretischen Boden zwischen dem 25. bis 50., bevorzugt zwischen dem 33. bis 40. theoretischen Boden.

12. Verfahren nach einem der Ansprüche 9 bis 11, **dadurch gekennzeichnet, dass** die Trennwand (8) in der Kolonne zwischen dem 10. und 60., bevorzugt zwischen dem 15. und 53. theoretischen Boden, bevorzugt mittig, angeordnet ist.

13. Verfahren nach einem der Ansprüche 9 bis 12, **dadurch gekennzeichnet, dass** als trennwirksame Einbauten geordnete Packungen oder Böden vorgesehen sind.

14. Verfahren nach Anspruch 13, **dadurch gekennzeichnet, dass** die Böden dergestalt ausgelegt sind, insbesondere bezüglich der Wehrhöhen, dass die Verweilzeit in der Kolonne 15 Minuten, bevorzugt 10 Minuten, nicht überschreitet.

## Claims

1. A process for obtaining pure 1,3-butadiene comprising at least 99.6% by weight of 1,3-butadiene, not more than 10 ppm of propyne and not more than 20 ppm of 1,2-butadiene from crude 1,3-butadiene comprising from about 89 to 99.5% by weight of 1,3-butadiene, balance impurities, by distillation, wherein the process is carried out in a dividing wall column (1) in which a dividing wall (8) is installed in the longitudinal direction of the column to form an upper common column region (9), a lower common column region (14), a feed section (10, 12) and an offtake section (11, 13) and the residence time in the bottom vaporizer (7) and the associated piping system is limited to from 1 to 15 minutes.

2. The process process according to claim 1, wherein the residence time in the bottom vaporizer (7) and the associated piping system is limited to from 3 to 6 minutes.

3. The process according to claim 1 or 2, wherein the liquid runback ratio at the upper end of the dividing wall (8) between the feed section (10) and the offtake section (11) of the column is set to a ratio of 1:1.3-2.2.

4. The process according to any of claims 1 to 3, wherein the ratio of the vapor flows at the lower end of the dividing wall (8) to the feed section (12) and the offtake section (13) of the column is set to a ratio of 1:0.7-1.3, preferably 1:0.95-1.1.

5. The process according to any of claims 1 to 4, wherein the pressure at the top of the column is from 2 to 10 bar, preferably from 4 to 7 bar.

6. The process according to any of claims 1 to 5, wherein the upper common column region is provided with a temperature regulator with a measurement point below the uppermost theoretical plate, preferably on the third theoretical plate from the top, which utilizes the distillate flow, the runback ratio or preferably the amount of runback as setting parameter.

7. The process according to any of claims 1 to 6, wherein the lower common column region is provided with a temperature regulator with a measurement point above the lowermost theoretical plate, preferably on the second theoretical plate from the bottom, which utilizes the amount taken off at the bottom as setting parameter.

8. The process according to any of claims 1 to 7, wherein the bottom of the column is provided with a level regulator which utilizes the amount taken off at the side offtake as setting parameter.

9. The process according to any of claims 1 to 8 which has from about 40 to 70, preferably from about 50 to 60, theoretical plates in the dividing wall column (1).

10. The process according to claim 9, wherein the feed point (2) for the crude 1,3-butadiene is located on a theoretical plate from the 20th to the 40th, preferably from the 25th to 35th, theoretical plate.

11. The process according to claim 9 or 10 which has a side offtake point (3) for the pure 1,3-butadiene at a theoretical plate from the 25th to the 50th, preferably from the 33rd to 40th, theoretical plate.

12. The process according to any of claims 9 to 11, wherein the dividing wall (8) is installed, preferably centrally, in the column between the 10th and 60th, preferably between the 15th and 53rd, theoretical plates.

13. The process according to any of claims 9 to 12 provided with ordered packing or trays as separation-active internals.

14. The process according to claim 13, wherein the trays are designed, particularly in respect of the weir heights, so that the residence time in the column does not exceed 15 minutes, preferably 10 minutes.

## Revendications

1. Procédé destiné à l'obtention par distillation de 1,3-butadiène pur, contenant au moins 99,6 % en poids de 1,3-butadiène, au maximum 10 ppm de propine et au maximum 20 ppm de 1,2-butadiène, à partir de 1,3-butadiène brut, contenant environ 89 à 99,5 % en poids de 1,3-butadiène, le reste représentant des impuretés, **caractérisé en ce que** l'on réalise le procédé dans une colonne à paroi séparatrice (1), dans laquelle une paroi séparatrice (8) est agencée dans la direction longitudinale de la colonne en formant une zone de colonne commune supérieure (9), une zone de colonne commune inférieure (14), une partie d'alimentation (10,12) et une partie de soutirage (11,13) et **en ce que** l'on limite le temps de séjour dans l'évaporateur de bas de colonne (7) et dans le système de conduite tubulaire correspondant à une durée de 1 à 15 minutes.

2. Procédé selon la revendication 1, **caractérisé en ce que** l'on limite le temps de séjour dans l'évaporateur de bas de colonne (7) et dans le système de conduite tubulaire correspondant à une durée de 3 à 6 minutes.

3. Procédé selon la revendication 1 ou 2, **caractérisé en ce que** l'on ajuste un rapport de reflux liquide de l'extrémité supérieure de la paroi séparatrice (8) à la partie d'alimentation (10) ou à la partie de soutirage (11) de la colonne de 1:1,3 à 2,2.

4. Procédé selon l'une quelconque des revendications 1 à 3, **caractérisé en ce que** l'on ajuste le rapport quantitatif des courants de fumées de l'extrémité inférieure de la paroi séparatrice (8) à la partie d'alimentation (12) ou à la partie de soutirage (13) de la colonne dans un rapport de 1:0,7 à 1,3, de préférence dans un rapport de 1:0,95 à 1,1.

5. Procédé selon l'une quelconque des revendications 1 à 4, **caractérisé par** une pression en tête de colonne de 2 à 10 bars, de préférence de 4 à 7 bars.

6. Procédé selon l'une quelconque des revendications 1 à 5, **caractérisé par** une régulation de température dans la zone de colonne commune supérieure, avec un point de mesure en dessous du plateau théorique supérieur, de préférence sur le 3^{ème} plateau théorique en partant du haut, laquelle régulation de température utilise le courant du distillat, le rapport de reflux ou, de préférence, la quantité de reflux comme grandeur de réglage.

7. Procédé selon l'une quelconque des revendications 1 à 6, **caractérisé par** une régulation de température dans la zone de colonne commune inférieure, avec un point de mesure au-dessus du plateau théorique inférieur, de préférence sur le 2^{ème} plateau théorique en partant du bas, laquelle régulation de température utilise la quantité soutirée du bas de colonne comme grandeur de réglage.

8. Procédé selon l'une quelconque des revendications 1 à 7, **caractérisé par** une régulation de niveau en bas de colonne qui utilise la quantité de soutirage latéral comme grandeur de réglage.

9. Procédé selon l'une quelconque des revendications 1 à 8, **caractérisé par** un nombre de plateaux théoriques d'environ 40 à 70, de préférence de 50 à 60 dans la colonne à paroi séparatrice (1).

10. Procédé selon la revendication 9, **caractérisé par** l'agencement du point d'alimentation (2) pour le 1,3-butadiène brut sur un plateau théorique situé entre les 20^{ème} et 40^{ème} plateaux théoriques, de préférence entre les 25^{ème} et 35^{ème} plateaux théoriques.

11. Procédé selon l'une quelconque des revendications 9 ou 10, **caractérisé par** un point de soutirage latéral (3) pour le 1,3-butadiène pur d'un plateau théorique situé entre les 25^{ème} et 50^{ème} plateaux théoriques, de préférence entre les 33^{ème} et 40^{ème} plateaux théoriques.

12. Procédé selon l'une quelconque des revendications 9 à 11, **caractérisé en ce que** la paroi séparatrice (8) est agencée dans la colonne entre les 10^{ème} et 60^{ème} plateaux théoriques, de préférence entre les 15^{ème} et 53^{ème} plateaux théoriques, de préférence au centre.

13. Procédé selon l'une quelconque des revendications 9 à 12, **caractérisé en ce que** l'on prévoit des garnissages ou des plateaux ordonnés comme éléments encastrés séparateurs.

14. Procédé selon la revendication 13, **caractérisé en ce que** les plateaux sont dimensionnés, en particulier concernant les hauteurs de déversoir, de sorte que le temps de séjour dans la colonne ne dépasse pas 15 minutes, de préférence 10 minutes.
